Europäisches Patentamt

(19) European Patent Office

Office européen des brevets

(11) Publication number: **0 161 000**
**A2**

# (12) EUROPEAN PATENT APPLICATION

(21) Application number: 85105740.6

(22) Date of filing: 10.05.85

(51) Int. Cl.⁴: **C 12 P 21/00**
**A 61 K 35/12, G 01 N 33/50**

(30) Priority: 11.05.84 US 609273

(43) Date of publication of application:
13.11.85 Bulletin 85/46

(84) Designated Contracting States:
DE FR GB

(71) Applicant: Sloan-Kettering Institute For Cancer Research
1275 York Avenue
New York New York 10021(US)

(72) Inventor: Platsoucas, Chris
504 East 81 Street
New York New York 10028(US)

(74) Representative: Patentanwälte Schulze Horn und Hoffmeister
Goldstrasse 36
D-4400 Münster(DE)

(54) Human suppressor factors and method of using them.

(57) New factors have been identified which suppress mitogen, antigen or alloantigen driven cellular proliferation of human peripheral blood leukocytes, as well as antibody synthesis and secretion and growth of human tumor cell lines.

Such factors have potential use for the treatment of graft versus host disease, autoimmuno disease and lymphoproliferative disorders such as leukemia as well as other malignancies.

Croydon Printing Company Ltd.

Human Suppressor Factors and Method

This invention relates to factor(s) which suppress cellular proliferation and antibody production and would be useful to control disorders such as those involving abnormal cellular proliferation.

## Description

Factor(s) has been identified which suppress not only mitogen or antigen (alloantigen) driven cellular proliferation of human peripheral blood leukocytes but also antibody production.

Such factor(s) have potential use for example in the treatment of patients with cancer, graft versus host disease (s), autoimmune disease(s) and lympho-proliferative malignancy disorder(s) such as leukemia.

Previous suppressor factors have been reported in the literature (See Grillot-Courvalin, Caterine et al., (1981) Nature 292:844; Greene, Warner C., et al. (1982) J. Immunol. 129:1986; M. J. Taussig, et al. (1979) Nature 277: at 305 and 308; Sirkka Kontianen, et al. (1978) Nature 274:477; Eisenthal, A., et al. (1979) Ann N. Y. Acad. Sci. 322:367; Smit, R. T., et al. (1970) Am. J. Pathol. 60:495; Namba, Y., et al. (1975) Inflammation 1:5; Lee, S. C., et al. (1977) J. Immunol. 118:88; Jegosothy, B. V., et al. (1979) J Exp. Med. 150:622; Namba, Y., et al. (1977) J. Immunol. 118:1379; Jegosathy, B. V., et al. (1976) 193:1260; and Waksman, B. H., et al. (1978) Cell Immunol. 36:1801.

The suppressor factor(s) described in the invention are different from all these factors because they: (a) exhibit different functional properties, (2) exhibit different molecular weight; (3) are produced constitutively and in substantially higher quantities.

Factor(s) are described and identified in this invention, which inhibit leukocyte proliferative responses. In particular these factor(s), supressor factor (SF), have been found to be produced by hematopoietic cells such as human lymphoblastoid cell lines - especially T cell lines and others such as B cell lines or erythroleukemic cell lines. Particular T cell lines known to produce supressor factors(s) are Jurkat, HPB-ALL, TALL-1, HD-MAR, SKW-3, DND 41, HPB-MLT and MOLT-4. Erytholeukemic cell lines such as K-562 can also produce SF. B cell lines such as CESS can also produce SF.

Supernatants from such cells inhibit the proliferative responses of T-lymphocytes to mitogens such as phytohaemagluttinin /PHA), concanavalin A (CON A) and pokeweed mitogen (PWM). (Tables I-III)

It also inhibits the proliferative responses of T-cells to allogeneic cells in mixed lymphocyte culture (MLC). (Table IV)

Also this factor(s) inhibits antibody production of blood cells, and expecially in vitro by human peripheral blood mononuclear leukocytes in the PWM-driven system (Table V). SF may inhibit certain proliferative responses of B-cells.

The action of SF Appears to be cytostatic not cytotoxic since it:

1. does not affect the viability of human peripheral blood mononuclear leukocytes in culture after a four day incubation (Table VI);

2. Does not cause lysis of K562 leukemic cell targets which can be determined by such assays as the chromium release assay; and

This factor(s) does not affect natural killer (NK) cell cytotoxicity against K-562 targets (Table VII)

Also this factor inhibits the growth in vitro of cells from certain human tumor cell lines (lung, colon, etc.) (Table VIII).

These SF(s) exhibit a relative molecular weight in the range of 55-70,000, as determined by AcA-44 gel filtration.

Table I - III shows the inhibitory effect of SF on the peripheral blood mononuclear leukocyte proliferative response to mitogens CON A, PWM and PHA. In these examples SF is produced by Jurkat, Molt-4, K-562 and HPB-ALL cells as well as other cell lines. In addition, hematopoietic cell hybridomas produce SF. Cess B cell line generally is positive for SF.

The examples illustrated in the tables are for illustrative purposes only and are not meant to limit the invention to the examples shown. It is noted that the invention is not limited to one single suppressor factor but it is believed that SF may be more than one molecule or

0161000

molecular form or, different cell lines produce different suppressor factors exhibiting similar or partially
similar properties.

SF can be useful in treating or preventing graft versus
host disease, also in patients who have received heterologous or autologous transplants whether of bone marrow,
kidney, heart et al. Also it would be useful in treating
autoimmune disease such as systemic lupus erythematosus,
myasthenia Gravis, Rheumatoid Arthritis, multiple Sclerosis and Allergies. SF has applications in the treatment of lympho-proliferative disorders such as leukemia
as well as in the treatment of other malignancies,
including human solid tumors such as lung, colon etc.

## Isolation of Peripheral Blood Mononuclear Leukocates Depletion of Monocytes

Mononuclear cells from normal donors were isolated by
centrifugation on a Ficoll/Hypaque density cushion
(Boyum, A. (1968) Scand. J. Clin. lab.Invest. 21:
(Suppl. 97) 77), at room temperature. The cells were
washed three times in Hank's balanced salt solution
(HBSS) and resuspended in RPMI-1640 containing 15 %
heat-inactivated fetal calf serum at a concentration of
$4 \times 10^6$ cells/ml. Lymphocyte separator reagent (Techni-
con Instrument Co., Tarrytown, NY) was added to the
mononuclear cell suspension at a volume ratio of 1 : 2
and the mixture was incubated at $37^o$ C on a rotator for
30 min. Phagocytic cells were depleted by subsequent
centrifugation at 400 x g for 20 min on a ficoll/hypaque
density cushion. Lymphoid cells depleted of phagocytic
cells were collected from the interface, washed three
time with Hank's balanced salt solution (HBSS) and re-
suspended at $4 \times 10^6$ cells/ml.

## Preparation of T lymphocytes

T lymphocytes were prepared by rosetting with neuraminidase-treated sheep erythrocytes (SRBC) (25 Units/ml of 5 % SRBC followed by centrifugation on ficoll/Hypaque as previously described (Platsoucas et al. (1980) J. Immunol. $\underline{125}$; 1216). Two-milliliter aliquots of lympho-cytes (4 x $10^6$/ml) in HBSS were mixed with 0.5 ml of heat-inactivated and SRBC-absorbed fetal calf serum and 2 ml of 1 % neuraminidase treated SRBC. The mixture was incubated for 5 min at $37^{\circ}$, centrifuged for 5 min at 200 X G, and incubated at $4^{\circ}$ C for an additional hour. The rosettes were resuspended carefully and incubated on ice for an additional 15 min. The cell suspensions were layered on a Ficoll/Hypaque density cushion and centrifuged at 400 x G for 20 min at controlled temperature ($22^{\circ}$ C). Non-T cells were recovered from the interface and were washed three times with HBSS. Rosetting T cells were recovered from the pellets after lysis of attached SRBC by Tris-buffered 0.83 % ammonium cloride /pH 7.2). The T cells were washed three times with HBSS. E-rosetting cells prepared by this method were more than 95 % T lymphocytes, as determined by rerosetting with SRBC without nonspecific esterase-positive cells and less than 2 % immunoglobulin-bearing cells. E-rosette-negative cells contained more than 70 % surface immunoglobulin cells, as determined by immunofluorescence, and less than 1 % of E-rosette forming cells or non-specific esterase positive cells. These cells were used as B cells. B cells are used for B cell growth factor assay.

## Proliferative Response to Mitogens

Human peripheral blood mononuclear leukocytes (at a concentration of 1 x $10^6$ cells/ml) were cultured in RPMI-1640 containing 10 % fetal calf serum and supplemented with 25 mM Hepes, 2mM L-glutamine and 100 units/ml Penicillin and 100 micrograms/ml streptomycin. One hundred microliters of the cell suspension were stimulated on U-microliter plates (Scientific Products) by various concentration of mitogens (PHA-P, Con A, PWM) at $37^O$ C in a humidified incubator in a 5 % $CO_2$, 95 % air environment. The cultures were pulsed with 25 microliters of tritiated thymidine (specific activity, 6.7 Ci/mmol, New England Nuclear, Boston, MA) after 72 hours and harvested using an automatic cell harvester 24 hours after the addition of the isotopes.

## Mixed Lymphocyte Culture

Human peripheral blood mononuclear leukocytes from various donors were prepared as above.

Responding cells ($1 \times 10^5$) were cultured with $1 \times 10^5$ stimulating cells in round bottom microtiter plates in total volume of 0.2 ml. The stimulating cells were inactivated by x-irradiation (2000 rads). The culture medium is RPMI-1640 supplemented with 10 % fetal calf serum, 25 mM Hepes, 2 mM L-glutamine, penicillin (100 units/ml) and streptomycin (100 microgram/ml). The cultures were incubated at $37^O$ C in a humidified atmosphere with 5 % $CO_2$, pulsed the 5th day with 1 micro Ci/well of $^3$H-thymidine (New England Nuclear, Boston, MA, specific activity 6.7 (Ci/mmole) and harvested 24 hours later on an automatic cell harvester (Skarton, Norway). All cultures were performed in quadruplicate.

## Natural Killer Cytotoxicity

Natural killer cytotoxicity was determined as previously described (platsoucas, et al. (1980) J. Immunol. 125:

1216). Target cells of the K562 and Molt-4 lines, maintained in RPMI-1640 supplemented with 10 % fetal calf serum, glutamine and antibiotics as above were labelled with 300 microliters of $^{51}$Cr per $2 \times 10^6$ cells (sodium ($^{51}$Cr) cromate, New England Nuclear, Boston, MA) for 2 hours. The target cells were washed three times and then resuspended in the same medium, at a concentration of $5 \times 10^4$ cells/ml. Effector lymphocytes were washed three times in RPMI-1640 supplemented with 10 % fetal calf serum, arranged at the appropriate concentration and one hundred microliters were added to one hundred microliters of target cells in U-bottom microtiter plates (Nunclon, Denmark), to achieve effector to target ratios 100 : 1, 50 : 1, and 25 : 1 etc. The plates were centrifuged at $40 \times g$ for 2 min and subsequently incubated at $37^\circ$C in a humidified 5 % $CO_2$ atmosphere. After 4 hours, the plates were centrifuged at $500 \times g$ for 5 min and 100 microliters of the supernatants were collected and counted for $^{51}$Cr release in a well-type Auto-Gamma scintillation counter.

Percent specific lysis is calculated by the formula:

$$\% \text{ Specific lysis} = \frac{E - S}{T - S} \times 100$$

were E = mean cpm released in the presence of effector cells. S = mean cpm spontaneously released by target cells incubated with medium alone, and T = mean cpm released after treating target cells with Triton x 100 (1 : 100 dilution).

## Cell Viability

Peripheral blood mononuclear leukocytes (MNL) were cul- tured at a concentration of $1 \times 10^6$ cells/ml in RPMI 1640 supplemented with 10 % heat-inactivated FCS, 25 mM herpes buffer, 2mM L-glutamine, and the antibiotics streptomycin 100 micrograms/ml and Pennicillin 100 U/ml for up to 88 hrs.

Control samples were incubated in the above medium and test samples are incubated with SF produced by Jurkat et al cell lines. Samples were withdrawn at 20 hrs, 44 hr, 68 hrs and 88 hrs. Cells were washed 2 x and viability was determined by Trypan blue dye exclusion.

<u>Origin of cells</u>

Cell lines used in the specification were established cell lines from patients with various leukemias.

<u>M. W. determinations:</u>

These were carried out by AcA-44 ultrogel (LKB) filtration in isotonic phosphate buffered saline (PBS). Molekular weight markers employed involved:

|  |  |
| --- | --- |
| Bovine serum albumin | 68,000 M.W. |
| Ovalbumin | 43,000 M.W. |
| Cytochrome c | 11,700 M.W. |

<u>Induction of de novo Ig synthesis and secretion by human peripheral blood mononuclear leukocytes in the PWM-induced differentiation system:</u>

Human peripheral blood mononuclear leukocytes were cultured at $1 \times 10^6$ cells/ml in RPMI-1640 supplemented with 10 % heat inactivated fetal calf serum, 2mM glutamine, Hepes and antibiotics as previously described, in total volume of 2 ml, for 7 days at $37^\circ$ C in 5 % $CO_2$ in a humidified incubator. Pokeweed mitogen (10 microgram /ml), optimal concentration; Grand Island Biological Co., Grand Island, NY) was added from the beginning of the culture. After incubation for 7 days the tube were centrifuged at 400 x g and supernatants were carefully withdrawn and stored at $-20^\circ$ C until assayed for immunoglobulin.

Determination of De Novo IgG, IgA, IgM Immunoglobulins by Enzyme-linked Immunoabsorbent Assay (ELISA)

These determinations were carried out by a modification of the method described by Engvall and Perlmann J. Immunol. 109:129 (1972). Rabbit anti-human immunoglobulin antibody, heavy chain specific (mu, gamma or alpha) (Accurate Chemical) were arranged at a concentration of 5 g/ml in 0.10 M $Na_2CO_3$, pH 9.6, containing 0.05 sodium azide. Two hundred microliters of antibody solution per well were transferred into 96-well round bottom microliter plates and incubated at $37^\circ$ C for 3 hours. The plates were stored at $4^\circ$ C until use and were stable for over two weeks. Bevore use the plates were washed with PBS containing 0.02 %. Tween 20 three times, were allowed to remain at room temperature for 5 min, between washings. Several dilutions of the unknown immunoglobulin containing supernatants were prepared in PBS containing 0.02 % Tween 20 and volumes of 0.2 ml will be transferred to the plates. The plates were incubated for 5 hours at room temperature, on a rocket platform.

Supernatants were removed by aspiration and the tubes were washed three times with PBS, containing 0.02 % Tween 20. Alkaline phosphatase conjugated rabbit anti-human immunoglobulin antibody heavy chain specific was obtained from AMF Immunoreagents Inc., Sequin, TX (gamma mu , or alpha heavy chain specific). Before use the conjugates were absorbed with 1 % ovalbumin solution in phosphate buffered saline (1 hour at room temperature), to absorb extra gluteraldhyde. One ml of conjugate diluted 1 : 500 with PBS-Tween 20, was added to the anti-human Ig-human Ig coated tubes, and the tubes were incubated for 16 hours at room temperature. Subsequently, the unbound conjugate was removed by washing the plates three times with PBS- Tween 20. The amount of bound alkaline phosphatase rabbit anti-human heavy chain specific, was determined using p-nitrophenylphosphate (NPP) (Sigma) as a substrate. One ml of 1 mg/ 1 NPP, in 0.05 M sodium carbonate buffer (pH 9.8) containing $10^{-3}$ M $MgCl_2$ was added to the plates and the released p-nitrophenolate was measured at 405 nm after one hour, using a titertek ELISA reader. Standard curves were constructed using purified IgG, IgA or IgM immunoglobulins for polyclonal immunoglobulin secretion, or purified paraproteins from patients with multiple myeloma for the determination of idiotypic immunoglobulin secretion.

PBS = phosphate buffered saline

Inhibition of the growth of human tumor cell lines by supernatants containing the SF:

Cells from human tumor lung lines were arranged at a concentration of $5 \times 10^4$ cells/ml, in Minimal Essential Medium supplemented with 10 % heat-inactivated fetal calf serum, 2 mM L-glutamine and antibiotics (streptomycin 100 microgram/ml and penicillin 100 U/ml). One ml of cell suspension was transferred to 24 well plates. Cultures were set up in triplicate. After the cells were attached, supernatants containing SF(s) were added. After 60 and 90 hours in culture, the cells were detached with EDTA (final concentration 0.2 % and counted.

0161000

TABLE IA

Inhibition of proliferative response of peripheral
blood mononuclear leukocytes to PHA, by SF produced
by the Jurkat, HPB-All and Molt 4 lines

| | PROLIFERATIVE RESPONSES (CPM) | | | | | |
|---|---|---|---|---|---|---|
| % SF (v/v) | Jurkat | | HPB-All | | Molt-4 | |
| | CPM | % suppression | CPM | % suppression | CPM | % suppression |
| medium | 513 ± 175 | — | 513 ± 175 | — | 513 ± 175 | — |
| medium + PHA | 11058 ± 1883 | — | 11058 ± 1883 | — | 11058 ± 1883 | — |
| medium + PHA + 0.015% SF | 2351 ± 223 | 79% | 6318 ± 1689 | 43% | 2399 ± 339 | 78% |
| medium + PHA + 0.05% SF | 2612 ± 337 | 76% | 2199 ± 1792 | 35% | 1118 ± 28 | 89% |
| medium + PHA + 0.1% SF | 2861 ± 269 | 74% | 8800 ± 2172 | 20% | 998 ± 105 | 91% |
| medium + PHA + 0.5% SF | 4404 ± 390 | 60% | 1778 ± 111 | 84% | 1333 ± 328 | 88% |
| medium + PHA + 0.95% SF | 3921 ± 277 | 65% | 1419 ± 234 | 87% | 1127 ± 77 | 89% |
| medium + PHA + 1.9% SF | 4156 ± 256 | 62% | 1564 ± 156 | 86% | 1130 ± 138 | 89% |
| medium + PHA + 3.75% SF | 4426 ± 354 | 60% | 2186 ± 265 | 80% | 1240 ± 82 | 89% |
| medium + PHA + 15% SF | 3512 ± 302 | 68% | 2335 ± 319 | 79% | 985 ± 36 | 91% |
| medium + PHA + 30% SF | 3538 ± 571 | 68% | 2685 ± 236 | 76% | 728 ± 57 | 93% |

Inhibition of proliferative response of peripheral
blood mononuclear leukocytes to PHA, by SF produced
by the K562, HPB-All, Molt 4 and CESS tumor cell lines

| % SF (v/v) | PROLIFERATIVE RESPONSES (CPM) | | | | | | | |
|---|---|---|---|---|---|---|---|---|
| | K562 | | Molt-4 | | Cess | | HPB-LL | |
| | CPM | % suppression | CPM | % suppression | CPM | % suppression | CPM | % suppression |
| medium | 2279 ± 14 | — | 2279 ± 14 | — | 2279 ± 14 | — | 2279 ± 14 | — |
| medium + PHA | 56211 ± 3811 | — | 56211 ± 3811 | — | 56211 ± 3811 | — | 56211 ± 3811 | — |
| medium + PHA + 1.87% SF | 1624 ± 88 | 97% | 1499 ± 248 | 97% | 48729 ± 4699 | 13% | 8124 ± 399 | 86% |
| medium + PHA + 3.75% SF | 1515 ± 190 | 97% | 1287 ± 130 | 98% | 51048 ± 6139 | 9% | 8109 ± 816 | 86% |
| medium + PHA + 7.5% SF | 2091 ± 942 | 96% | 1249 ± 132 | 98% | 53579 ± 3152 | 5% | 6687 ± 773 | 88% |
| medium + PHA + 15% SF | 1536 ± 119 | 97% | 1225 ± 143 | 98% | 59898 ± 5211 | 6% | 7916 ± 1116 | 86% |
| medium + PHA + 30% SF | 2009 ± 254 | 96% | 865 ± 101 | 98% | 31823 ± 1550 | 43% | 5434 ± 777 | 90% |

## TABLE 1C

Inhibition of proliferative response of human peripheral
blood mononuclear leukocytes to PHA, by SF produced
by TALL-1, HD-Mar, SKW-3, DND-41 and HPB-MLT

|  | Cell line Supernatant** | CPM | % Suppression |
|---|---|---|---|
| MNC* | None | 4677 ± 1367 | |
| MNC + PHA | None | 93646 ± 9369 | |
| MNC + PHA | TALL-1 | 18119 ± 3822 | 81 |
| MNC + PHA | HD-Mar | 9914 ± 1182 | 89 |
| MNC + PHA | SKW-3 | 9268 ± 434 | 90 |
| MNC + PHA | DND-41 | 28633 ± 3356 | 69 |
| MNC + PHA | HPB-MLT | 12136 ± 342 | 87 |

*MNC: Human, peripheral blood mononuclear leukocytes.

** Cell line supernatants were employed at concentration of 25% (v/v).

TABLE II

Inhibition of proliferative response of peripheral
blood mononuclear leukocytes to CON A , by SF produced
by the Jurkat, HPB-All and Molt 4 lines

PROLIFERATIVE RESPONSES (CPM)

| % SF (v/v) | Jurkat | | HPB-All | | Molt-4 | |
|---|---|---|---|---|---|---|
| | CPM | % suppre-ssion | CPM | % suppre-ssion | CPM | % suppre-ssion |
| medium | 265 ± 47 | — | 265 ± 47 | — | 265 ± 47 | — |
| medium + CON A | 40792 ± 7959 | — | 40792 ± 7454 | — | 40792 ± 7454 | — |
| medium + CON A + 0.015% SF | 3916 ± 239 | 90% | 61751 ± 13610 | — | 14440 ± 6966 | 65% |
| medium + CON A + 0.05% SF | 3606 ± 379 | 91% | 31713 ± 11104 | 22% | 4814 ± 580 | 88% |
| medium + CON A + 0.1% SF | 3889 ± 413 | 90% | 12399 ± 2129 | 69% | 4126 ± 681 | 90% |
| medium + CON A + 0.5% SF | 4489 ± 649 | 89% | 6414 ± 1356 | 84% | 3233 ± 688 | 92% |
| medium + CON A + 0.95% SF | 3671 ± 294 | 91% | 3886 ± 1055 | 90% | 2718 ± 910 | 93% |
| medium + CON A + 1.9% SF | 3615 ± 211 | 91% | 3678 ± 765 | 91% | 2244 ± 446 | 94% |
| medium + CON A + 3.75% SF | 3649 ± 350 | 91% | 3879 ± 734 | 90% | 26622 ± 401 | 93% |
| medium + CON A + 15% SF | 3605 ± 191 | 91% | 3421 ± 404 | 92% | 1729 ± 108 | 96% |
| medium + CON A + 30% SF | 3862 ± 381 | 91% | 3637 ± 296 | 91% | 855 ± 48 | 98% |

TABLE III

Inhibition of proliferative response of peripheral
blood mononuclear leukocytes to PWM by SF produced
by the Jurkat tumor cell line

| % SF (v/v) | PROLIFERATIVE RESPONSES (CPM) | |
| --- | --- | --- |
| | Jurkat | |
| | CPM | % suppression |
| Medium | 703 ± 84 | — |
| Medium + PWM | 13119 ± 1792 | — |
| Medium + PWM + 1.56% SF | 4631 ± 389 | 65% |
| Medium + PWM + 3.12% SF | 4560 ± 373 | 65% |
| Medium + PWM + 6.25% SF | 3624 ± 381 | 72% |
| Medium + PWM + 12.5% SF | 4474 ± 306 | 66% |
| Medium + PWM + 25% SF | 5095 ± 352 | 61% |

## TABLE IV

Inhibition by SF produced by the Jurkat tumor
cell lines, of the proliferative responses of human
peripheral blood mononuclear leukocytes to allogeneic cells in
mixed lymphocyte culture

| % SF (v/v) | PROLIFERATIVE RESPONSES (CPM) | |
| --- | --- | --- |
| | Jurkat | |
| | CPM | % suppression |
| Medium | 7382 | -- |
| Medium + 1.56% SF | 3849 | 48% |
| Medium + 3.12% SF | 3229 | 56% |
| Medium + 6.25% SF | 3922 | 47% |
| Medium + 12.50% SF | 3678 | 50% |
| Medium + 25.00% SF | 6159 | 17% |

## TABLE V

Inhibition of de novo immunoglobulin synthesis and secretion of human peripheral blood mononuclear leukocytes in the PWM-induced differentiation system, by SF produced by the Jurkat, HPB-ALL, Molt-4, K562 and CESS CELL LINES.

| | Cell line Supernatants Dilutions | Immunoglobulin* | | |
|---|---|---|---|---|
| | | IgM (microg/dl) | IgA (microg/dl) | IgG (microg/dl) |
| Mononuclear cells + PWM | None | 243.0 | 70.5 | 54.3 |
| | Jurkat | | | |
| MNC + PWM | 1:3 | 19.0 | l.t. 2.0 | l.t. 2.0 |
| MNC + PWM | 1:50 | 20.5 | l.t. 2.0 | ND |
| MNC + PWM | 1:1000 | 15.0 | 3.75 | ND |
| MNC + PWM | 1:10000 | 19.0 | 4.5 | ND |
| | HPB-ALL | | | |
| MNC + PWM | 1:3 | 10.5 | l.t. 2.0 | l.t. 2.0 |
| MNC + PWM | 1:50 | 35.0 | 5.7 | l.t. 2.0 |
| MNC + PWM | 1:1000 | 27.3 | 11.3 | l.t. 2.0 |
| MNC + PWM | 1:10000 | 22.3 | 13.0 | l.t. 2.0 |
| | Molt-4 | | | |
| MNC + PWM | 1:3 | 23.5 | l.t. 2.0 | ND |
| MNC + PWM | 1:50 | 36.7 | 14.3 | l.t. 2.0 |
| MNC + PWM | 1:1000 | 35.7 | 16.0 | l.t. 2.0 |
| | K562 | | | |
| MNC + PWM | 1:3 | 9.9 | l.t. 2.0 | ND |
| MNC + PWM | 1:50 | 17.8 | l.t. 2.0 | l:t. 2.0 |
| MNC + PWM | 1:1000 | 18.3 | 9.0 | ND |
| | CESS | | | |
| MNC + PWM | 1:3 | 12.2 | l.t. 2.0 | 10.3 |
| MNC + PWM | 1:50 | 18.0 | l.t. 2.0 | ND |
| MNC + PWM | 1:1000 | 18.3 | 11.2 | ND |

*Determined by ELISA
ND - not determined
l.t. - less than

## TABLE VI

The viability of peripheral blood mononuclear
leukocytes is not affected by prolonged
incubation with SF produced by the Jurkat*
tumor cell lines

| Tumor cell lines | % Viability | | | |
| --- | --- | --- | --- | --- |
| | Duration of treatment | | | |
| | 20 hrs | 44 hrs | 68 hrs | 88 hrs |
| Medium * alone | 98% | 100% | 96% | 93% |
| Jurkat | 99% | 96% | 95% | 90% |

*This effect is also seen with HPB-ALL, K562 and Molt-4 supernatants.

0161000

TABLE VII

Suppressor factor(s) preparations produced by the Jurkat
cell lines do not affect natural killer cytotoxicity: mediated by
peripheral blood mononuclear leukocytes, against K562 targets

| SF Source | % Cytotoxicity | | | |
|-----------|------|------|------|------|
| | Effector to target ratio | | | |
| | Donor 1 | | Donor 2 | |
| | 50:1 | 25:1 | 50:1 | 25:1 |
| Medium | 66 | 59 | 58 | 50 |
| Jurkat | 68 | 56 | 51 | 40 |

## TABLE VIII

Inhibition of the growth of human lung tumor cell lines by SF - containing supernatants form the Jurkat, HPB-ALL, K562 and Molt-4 human tumor cell lines

| | Cell Numbers | | | | | |
|---|---|---|---|---|---|---|
| Cell line | Lines | | | | | |
| | SK-LC-6 | | | SK-LC-14 | | |
| Supernatants* | Hours of Treatment | | | Hours of Treatment | | |
| | 0 | 60 | 90 | 0 | 60 | 90 |
| None | $0.5 \times 10^5$ | $1.4 \times 10^5$ | $5.7 \times 10^5$ | $0.5 \times 10^5$ | $1.7 \times 10^5$ | $4.2 \times 10^5$ |
| Jurkat | $0.5 \times 10^5$ | $0.6 \times 10^5$ | $1.5 \times 10^5$ | $0.5 \times 10^5$ | $1.4 \times 10^5$ | $1.9 \times 10^5$ |
| HPB-All | $0.5 \times 10^5$ | $1.1 \times 10^5$ | $1.4 \times 10^5$ | $0.5 \times 10^5$ | $2.1 \times 10^5$ | $3.0 \times 10^5$ |
| K562 | $0.5 \times 10^5$ | $0.6 \times 10^5$ | $1.2 \times 10^5$ | $0.5 \times 10^5$ | $0.7 \times 10^5$ | $2.0 \times 10^5$ |
| Molt-4 | $0.5 \times 10^5$ | $0.7 \times 10^5$ | $2.7 \times 10^5$ | | ND | |

*These were used at a dilution of 30% (v/v).

A1

What is Claimed:

1. Human suppressor factor characterized by suppression of mitogen or antigen driven cellular proliferation of human peripheral blood leukocytes and suppression of antibody production.

2. Human suppressor factor of Claim 1 as produced by human tumor cells or cell lines.

3. Suppressor factor of Claim 1 as produced by human hematopoietic cells or cell lines.

4. Human suppressor factor of claim 1 of relative molecular weight 55-70,000 daltons.

5. Suppressor factor of Claim 2 as produced by human erythroleukemic cells in culture.

6. Suppressor factor of Claim 4 as produced by human lymphoblastoid cells.

7. Suppressor factor of Claim 6 as produced by human T cells.

8. Suppressor factor of Claim 6 as produced by human B cells.

9. Suppressor factor of Claim 1 which suppresses cellular proliferative response.

10. Suppressor factor of Claim 9 which suppresses T-cell proliferative response.

11. Suppressor factor of Claim 9 which suppresses B-cell proliferative response.

12. Suppressor factor of Claim 9 which suppresses cellular proliferative response driven by alloantigens, antigens and mitogens selected from the group consisting of PWM, Con A, PHA, and mixtures thereof.

13. Suppressor factor of Claim 1 which suppresses antibody synthesis and secretion.

14. Suppressor factor of Claim 1 which inhibits proliferative response of human peripheral blood mononuclear leukocytes to allogeneic cells in mixed lymphocyte cultures.

15. Suppressor factor of Claim 1 which inhibits the proliferation of human tumor cell lines.

16. Supressor factor of Claim 1 which inhibits antibody production in vitro by human peripheral blood mononuclear leukocytes exposed to PWM.

17. Suppressor factor of Claim 1 which does not affect the viability of human peripheral blood mononuclear leukocytes in culture.

18. Suppressor factor of Claim 1 which does not affect natural killer cytotoxicity against K-562 targets.

19. Suppressor factor of Claim 1 which does not cause lysis of K-562 leikemic cell targets as determined by the chromium release assay.

20. Method for characterization of a suppressor factor which comprises exposing mitogen treated human peripheral blood mononuclear leukocyte specimens to a hematopoietic cell specimen and observing the cellular proliferative response or lack thereof of any of Claims 9 - 19.

21. Method of Claim 20 wherein the mitogen is selected from the class consisting of CON A, PWM, PHA and mixtures thereof.

22. Human suppressor factor of claim 1 for treatment of leukemia by exposing leukemic cells in vitro or in vivo to effective amounts of SF.

23. Human suppressor factor of claim 1 for treating or preventing graft versus host disease.

24. Human suppressor factor of claim 1 for the treatment of human malignancy.

26. Human suppressor factor of claim 1 for the treatment of transplantation disorders.

27. Human suppressor factor of claim 1 for the treatment of auto-immune disease selcted from the group consisting of Rheumatoid Arthritis, systemic lupus erythematosus, myasthemia gravis and multiple sclerosis and allergies.

25. Human suppressor factor of claim 1 for the treatment of human lymphoma or leukemia.